# EUROPEAN PATENT SPECIFICATION

(11) **EP 2 959 851 B1**
(45) Date of publication and mention of the grant of the patent: **25.01.2017**
(21) Application number: 15173851.5
(22) Date of filing: 25.06.2015
(51) Int. Cl.: A61B 17/16

(54) **FLEXIBLE INTRAMEDULLARY REAMER**
BIEGSAME INTRAMEDULLÄRE REIBAHLE
ALÉSOIR INTRAMÉDULLAIRE FLEXIBLE

(30) Priority: 27.06.2014 IT MI20141179
(43) Date of publication of application: 30.12.2015
(73) Proprietor: Technosprings Italia S.r.l., 21010 Besnate (VA) (IT); SIMEX Medizintechnik GmbH, 78652 Dreisslingen-Lauffen (DE); Fricweld AB, 71230 Hallefors (SE)
(72) Inventor: STORTIERO, Francesco, 20099 Sesto San Giovanni (MI) (IT); GUALANDRIS, Stefano, 6565 Mesocco (CH); SIRING, Rainer, 78652 Deißlingen (DE); SANDBERG, Dan, 68131 Kristinehamn (SE); ANDERS, Christian, Cirencester, Gloucestershire GL7 1YJ (GB); HEKMAN, Edsko E. G., 7500AE Enschede (NL); KOOPMAN, Hart F. J. M., 7500AE Enschede (NL); HAARMAN, Claudia J. W., 7500AE Enschede (NL)
(74) Representative: Mittler, Enrico

(56) References cited:
- EP-A1- 0 253 526
- DE-A1-102012 211 481
- GB-A- 1 360 221
- JP-A- S58 160 008
- US-A1- 2008 140 078

## Description

The present invention relates to a flexible intramedullary reamer.

Patent EP-253526-B1 describes a flexible intramedullary reamer comprising a flexible shaft. The flexible shaft is a hollow tube made of thermoplastics, thermosetting or fibre-reinforced composite material.

Patent US-4751922 of the same family of EP-253526-B1 describes that the flexible shaft is made of a material from the group consisting of carbon fiber composites. At an end of the shaft is connected a cutting head.

Disadvantageously the connection between the flexible shaft and the cutting head can have problems of contamination due to crevices in the interlayer used in welding process. Crevices are critical to clean in post-surgical process. In the crevices can lurk microbes and bacteria. Said problems of contamination are critical to avoid cross infection between patients. Such infections are particular concern in post-surgery patients, causing also death.

Disadvantageously the flexible shaft is made of low bio-compatible materials and contact with the biological materials must be limited in time. After a few cycles of sterilization that usually occur at about 135 degrees Celsius, the material of the flexible intramedullary reamer is very degraded and fragile, so that in case of breakage it produces a considerable number of fragments that can cause death to the patient.

Further disadvange is that laser welding technique produces crevices in the structures of the flexible intramedullary reamer, causing cross infection between patients.

Patent US-8518044-B2 describes a flexible intramedullary reamer comprising a flexible shaft made of Nitinol and formed from a double wound wire spiral which can disadvantageously have problems of contamination critical to avoid cross infection between patients because in the interstices of the wires can lurk microbes and bacteria. Said interstices are critical to clean in post-surgical process.

Patent US-5499984-A describes a flexible intramedullary reamer comprising a flexible shaft preferably made of nickel-titanium alloy. The flexible shaft is hollow. One end of the shaft comprise a male connector connecting by a female connector of a cutting head. The cutting head is preferably made of stainless steel and it is separably connected with the flexible shaft.

Disadvantageously the connection between the shaft and the cutting head is not reliable and can break off, therefore the cutting head or parts of it can remain into the bone causing also the death of the patient.

Prior art describes shafts preferably made of nickel-titanium alloy to reach such a flexibility to adapt the shaft to the bone, instead cutting hedges are made of stainless steel to reach better performances.

Patent US-2008/0140078-A1 describes a flexible reamer where the reamer head is laser-welded to the hollow shaft which is made of nitinol or elastic hardened steel.

It is not recommended to weld together a portion of TiNi alloy and a portion of stainless steel to avoid a large amount of brittle intermetallic compound formed at the weld interface causing the joint embrittlement which led to the degradation of the joint strength causing a break off unsafe for the patients.

Interlayer commonly used in welding process are not bio-compatible causing surgical and post-surgical critical problems for patients.

Bio-compatible materials are ideals for most severe corrosion situations such as surgical situations but have too low tensile strength discouraging skilled person who would think that danger of break off between the cutting head and the shaft.

The object of the present invention is to make a flexible intramedullary reamer crevice free, highly reliable surgical instrument, bio-compatible, high tensile strength, safe for patient, easy to clean and maintain.

In accordance with the invention, this object is achieved with a flexible intramedullary reamer comprising a flexible shaft made of shape memory alloy and a cutting head welded together, characterized in that said flexible shaft is welded together with said cutting head by means of an interlayer made of Tantalum.

These and other features of the present invention will become increasingly apparent from the following detailed description of one of its non-limiting practical embodiment examples disclosed in the accompanying drawings, in which:
figure 1 shows a side view of flexible intramedullary reamer comprising a flexible shaft made of Nitinol, an interlayer made of pure Tantalum and a cutting head made of stainless steel welded together;
figure 2 shows a sectional view of said flexible intramedullary reamer according to the line II-II in figure 1;
figure 3 shows an end portion of the flexible shaft made of Nitinol before welding process;
figure 4 shows an end portion of the interlayer made of pure Tantalum before welding process;
figure 5 shows a sectional view according to the line V-V in figure 3;
figure 6 shows a sectional view according to the line VI-VI in figure 4;
figure 7 shows an end portion of the flexible shaft made of Nitinol welded together with interlayer of pure Tantalum;
figure 8 shows an end portion of the cutting head made of stainless steel before welding process;
figure 9 shows a sectional view according to the line IX-IX in figure 7;
figure 10 shows a sectional view according to the line X-X in figure 8.

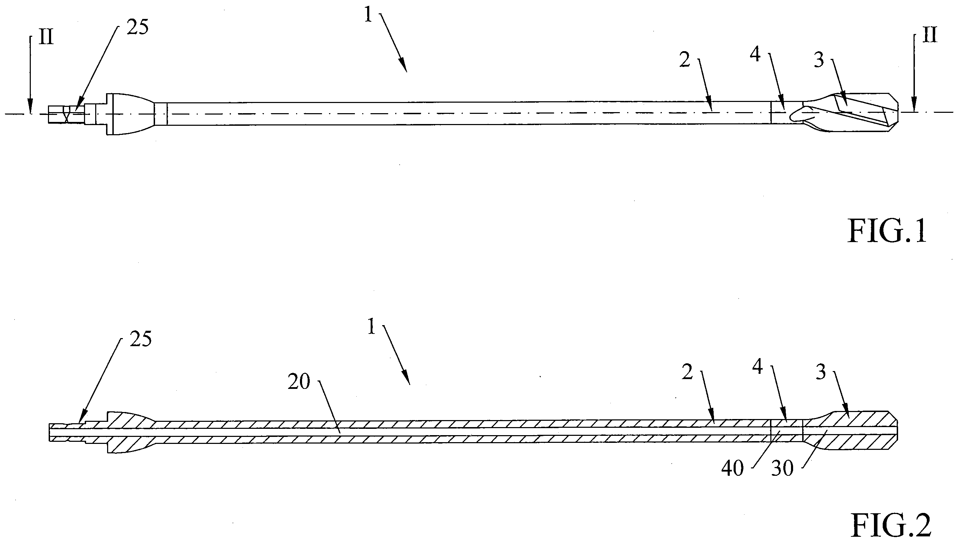

With reference to the above-listed figures, a flexible intramedullary reamer 1 for surgical operations to bones, comprises a flexible shaft 2 made of shape memory alloy and a cutting head 3 welded together by means of an interlayer rod 4 made of Tantalum, as shown in figure 1.

Said flexible intramedullary reamer 1 is suitable for being an instrument used for the treatment of the medullary space of bones.

The cutting head 3 is at one end of the flexible intramedullary reamer 1.

The other end of the flexible shaft 2 comprises an adapter 25 for the connection of the flexible shaft 2 to a power drive mechanism thereby to cause rotation of the flexible shaft 2 and the cutting head 3. Said flexible shaft 2 is rotatable. The flexible shaft 2 is a monolithic flexible shaft 2 without dangerous interstices where microbes or bacteria could lurking.

In the treatment of the medullary space of bones flexible intramedullary reamer 1 is used as a power-driven reamer for clearing, enlarging and reaming the medullary space of bones.

The joint between the cutting head 3 and the flexible shaft 2 has to support high tensile strength and high torsional strength.

The flexible shaft 2 is made of shape memory alloy (SMA hereinafter) such as Nickel Titanum alloy, because SMA has unique thermo-mechanical properties such as a shape memory effect (SME hereinafter) and a superelasticity (SE hereinafter) thanks to the martensitic transformation. Said transformation is an allotropic phase change wchich involves a martensitic (M hereinafter) and austenitic (A hereinafter) phase, the former stable a temperature below the latter.

Thus the SME phenomenon is related the recovery of the shape of a item made from SMA which can be deformed at stable Martensitic temperature maintaining the shape until it is heated up to the stable Austenitic temperature recovering the original shape. The transformation from M to A is promoted by temperature change.

The SE phenomenon is due to the reverse transformation from A to M promoted by load application and in case of an item made from SMA with a stable Austenitic temperature near to Body or Room temperature it is possible to deform it applying a load causing deformation up to 8% and when load is released the item recovers the original shape without any permanent deformation. The SE is often exploited to manufacture object that have to be undergone to severe deformation without reaching rupture or presenting plastic deformation.

Furthermore, during the loading and unloading process the mechanical behaviour of the SMAs is hysteretic so the mechanical hysteresis of SE elements is able to dissipate mechanical energy into heat allowing to be used as dissipative devices.

The SE and intrinsic dissipative properties of SMAs provides an advantage for making flexible intramedullary reamer allowing to the shaft to experiment large deformation without breaking and to dissipate vibrations due to the drilling process reducing the possibility of blocking of the reamer reducing the number of steps to reach final hole diameter and increasing the precision of the same hole.

This phenomenon provides an advantage for making flexible intramedullary reamer 1. The most common shape memory material is an alloy of nickel and titanium called Nitinol, but it is provided to use also other SMAs.

In particular Nitinol has very good mechanical properties, long fatigue life, and high corrosion resistance in severe corrosion situations such as surgical situations. Nitinol also has resistance properties. In most cases, the transition temperature of the SMA is chosen such that room temperature is well below the transformation point of the material.

Nitinol is preferably conforming to ATM F2063-05 standard.

The cutting head 3 is made of stainless steel to drill into marrow of long bone, such as femur or short bones or other bones in general. Stainless steel is safe for patients and reliable to the cutting head 3 avoiding its breaking and avoiding breaking into fragments dangerous for patients.

The stainless steel is preferably a 17-4PH (H900) stainless steel conforming to ASTM A564 standard.

The welding interlayer rod 4, between a portion of the flexible shaft 2 and a portion of the cutting head 3, is made of Tantalum.

Preferably Tantalum is a pure Tantalum conforming to ATM F560-08 (R05-200) standard, fully annealed, preferred maximum hardness of 88 HV to reach the better technical effect when used for interlayer according to the present invention.

Tantalum is ideals for most severe corrosion situations such as surgical situations, in fact Tantalum is used in surgical application, but have too low tensile strength of only 230-250 Mpa for fully annealed bars. It discourages any skilled person to use Tantalum as an interlayer for welding.

Many skilled persons use interlayers in Nickel which yields a tensile strength of 512 Mpa, but the use of interlayer made of Nickel is dangerous, because at the weld interface there is a large amount of intermetallic compounds formed causing crevices unsafe for patient. The formation of said compounds led to embrittlement of the joint strength causing break off unsafe for the patients.

It is an unexpected surprise that the technical effect of interlayer rod 4 made of Tantalum welded by means of rotary friction welding yields a tensile strength of 700 Mpa, advantageously higher than interlayer in Nickel and advantageously bio-compatible. Advantageously the interlayer rod 4 made of Tantalum can resist to high tensile strength and to high torsional strength.

Further Tantalum has mechanical properties advantageously superior with respect to other bio-compatible materials such as gold or silver.

As shown in figure 2 the flexible intramedullary reamer 1 comprise a through hole along its longitudinal axis.

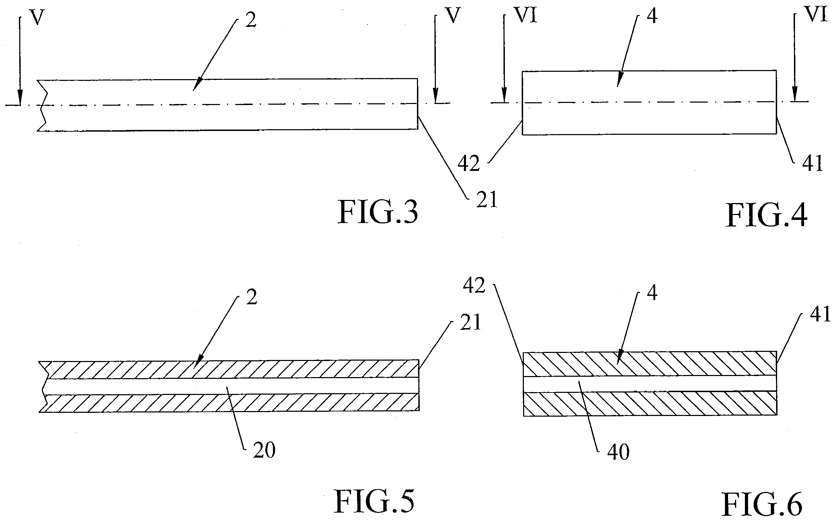

As shown in figures 3-10 the flexible shaft 2, the interlayer 4 and the cutting head 3 are hollow tubes. The through hole of the flexible intramedullary reamer 1 comprising a through hole 20 of the flexible shaft 2, a through hole 40 of the interlayer 4 and a through hole 30 of the cutting head 3 aligned together along said longitudinal axis and having same measures adapted for a guidewire suitable for passing through in surgical operation.

As shown in figures 3 and 5 the flexible shaft 3 is a tube and comprises an end portion 21 with a flat surface.

As shown in figures 4 and 6 the interlayer 4 is a tube and comprises a first end portion 42 with a flat surface and on the other side a second end portion 41 with a flat surface.

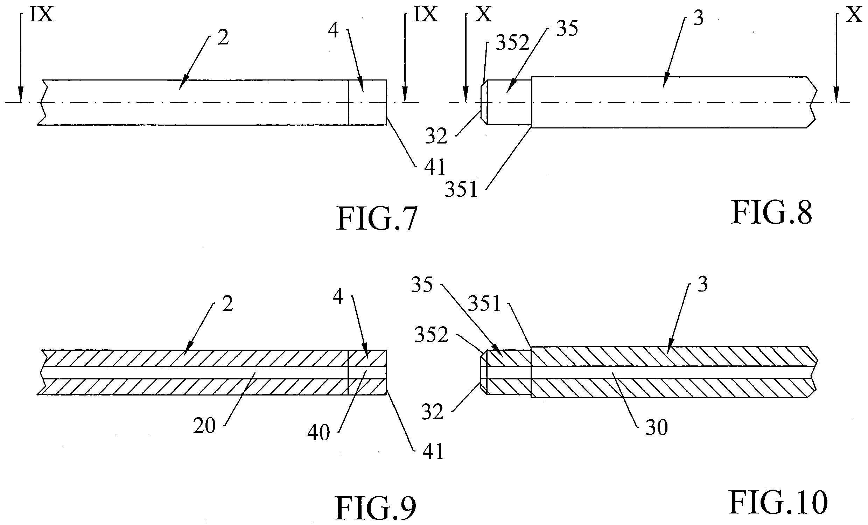

As shown in figures 8 and 10 the cutting head 3 is a tube and comprises an end portion 32 with a flat surface.

All the surfaces of the end portions 21, 42, 41, 32 are finished better than 3.2 micrometer by means of a mechanical machine to avoid advantageously weld face mis-match and to avoid advantageously angular deviation.

As shown in figures 8 and 10, at the end portion 32 of the cutting head 3 the measure of diameter of the end portion 32 is reduced by creating a portion 35 comprising a shoulder 351 and a chamfering 352 to provide an invitation to the second end 41 portion of interlayer 4. The portion 35, the shoulder 351 and the chamfering 352 are advantageous for welding the cutting head 3 and a welded group 2, 4.

With regards to the operation to make said flexible intramedullary reamer 1, a method to joint together said flexible shaft 2 made of shape memory alloy and said cutting head 3 of a flexible intramedullary reamer 1 comprises a welding phase to weld said flexible shaft 2 with said cutting head 3 by means of an interlayer 4 made of Tantalum.

Said welding phase comprises at least two steps.

Before a first welding step it is provided a first preparatory step to make flat the surface of the first end 42 of said interlayer 4 and to make flat the surface of the end 21 of said flexible shaft 2.

After the first preparatory step it is provided a first cleaning step to clean with a decontamination solution, such as acetone, the surface of the first end 42 of said interlayer 4 and the surface of the end 21 of said flexible shaft 2.

The first welding step is for welding said first end portion 42 of said interlayer 4 by means of rotary friction welding with said end portion 21 of said flexible shaft 2.

Said first welding step forms the welded group 2, 4 consisting of said flexible shaft 2 welded together with said interlayer 4.

A second welding step is successive to said first welding step, but before the second welding step it is provided a second preparatory step to make flat the surface of the second end portion 41 of said interlayer 4 and to make flat the surface of the end portion 32 of said cutting head 3.

The second preparatory step provides a further step to reduce the measure of diameter of the end portion 32 by creating a portion 35 comprising a shoulder 351 and a chamfering 352 to provide an invitation to the second end 41 portion of interlayer 4 which is advantageous for the second welding step.

After the second preparatory step it is provided a second cleaning step to clean with said decontamination solution the surface of the second end 41 of said interlayer 4 and the surface of the end 32 of said cutting head 3.

Said second welding step for welding said second end portion 41 of said interlayer 4 by means of rotary friction welding with said end portion 32 of said cutting head 3.

Rotary friction welding techniques are know in the prior art.

The means of rotary friction welding comprise a machine which comprises a rotary chuck suitable to rotate a portion with respect to an other portion. It generates heat through mechanical friction between said rotating portion and a stationary portion. No melt occurs, so the flexible shaft 2 made of Nitinol does not deform itself.

In the first welding step the Nitinol portion of flexible shaft 2 is mounted with a static clamp. Tantalum portion of the interlayer 4 is mounted with said rotary chuck.

In the second welding step the Nitinol-Tantalum portion of the welded group 2, 4 is mounted with said static clamp. Stainless steel portion of the cutting head 3 is mounted with said rotary chuck.

Revolution speed of said rotary chuck is set preferably to 3500 revolutions/minute to obtain a better result.

After the first welding step, it is provided a first removing step, and after the second welding step, it is provided a second removing step.

Rotary friction welding process produces welding burrs, such as upset and flash that needs to be removed.

Said first and second removing steps suitable for removing by mechanical means said welding burrs from welding portions of the flexible intramedullary reamer 1.

Alternatively the flexible shaft 2, the interlayer 4 and the cutting head 3 are solid bars without through holes.

A further alternative provides that said first welding step is successive to said second welding step.

Advantageously rotary friction welding process synergistically combined with the interlayer of Tantalum successfully provides flexible intramedullary reamer 1 with maximum stress level and highly reliable surgical instrument, all the materials of the interlayer 4, the cutting head 3 and the flexible shaft 2 are bio-compatible. The flexible intramedullary reamer has an advantageusly high tensile strength, also in the joint welding portions. Welding portions and all the portions of the flexible intramedullary reamer 1 are safe for patient, crevice free, easy to clean and maintain.

Advantageously the interlayer 4 of Tantalum welded to Nitnol and to stainless steel by means of rotary friction welding, provides strong, resistance and resilient welds between stainless steel and Nitinol.

## Claims

1. A flexible intramedullary reamer (1) comprising a flexible shaft (2) made of shape memory alloy and a cutting head (3) welded together, **characterized in that** said flexible shaft (2) is welded together with said cutting head (3) by means of an interlayer (4) made of Tantalum.

2. A flexible intramedullary reamer (1) according to claim 1, **characterized in that** the flexible shaft (2) is made of Nickel Titanum alloy, the cutting head (3) is made of stainless steel and the interlayer (4) is made of pure Tantalum.

3. A flexible intramedullary reamer (1) according to claims 1 or 2, **characterized in that** the flexible shaft (2), the interlayer (4) and the cutting head (3) are hollow tubes comprising a through hole (20) of the flexible shaft (2), a through hole (40) of the interlayer (4) and a through hole (30) of the cutting head (3) aligned together and having same measures adapted for a guidewire suitable for passing through.

4. Method to join together a flexible shaft (2) made of shape memory alloy and a cutting head (3) of a flexible intramedullary reamer (1), **characterized in that** comprises a welding phase to weld said flexible shaft (2) with said cutting head (3) by means of an interlayer (4) made of Tantalum.

5. Method according to claim 4, **characterized in that** said welding phase comprises at least two steps,
a first welding step is for welding a first end portion (42) of said interlayer (4) by means of rotary friction welding with an end portion (21) of said flexible shaft (2), and
a second welding step is for welding a second end portion (41) of said interlayer (4) by means of rotary friction welding with an end portion (32) of said cutting head (3).

6. Method according to claim 5, **characterized in that**
said first welding step forms a first welded group (2, 4) consisting of said flexible shaft (2) welded together with said interlayer (4) and
said second welding step is successive to said first welding step.

7. Method according to the claims 5 or 6, **characterized in that**
before the first welding step it is provided a first preparatory step to make flat a surface of the first end portion (42) of said interlayer (4) and to make flat a surface of the end portion (21) of said flexible shaft (2), and
before the second welding step it is provided a second preparatory step to make flat a surface of the second end portion (41) of said interlayer (4) and to make flat a surface of the end portion (32) of said cutting head (3).

8. Method according to claim 7, **characterized in that** after the first preparatory step it is provided a first cleaning step to clean with a decontamination solution the surface of the first end portion (42) of said interlayer (4) and the surface of the end portion (21) of said flexible shaft (2), and
after the second preparatory step it is provided a second cleaning step to clean with said decontamination solution the surface of the second end portion (41) of said interlayer (4) and the surface of the end portion (32) of said cutting head (3).

9. Method according to anyone of the preceding claims 5-8, **characterized in that**
after the first welding step, it is provided a first removing step, and
after the second welding step, it is provided a second removing step,
said first and second removing steps are suitable for removing by mechanical means welding burrs from welding portions of the flexible intramedullary reamer (1).

10. Method according to anyone of the preceding claims 4-9, **characterized in that** the flexible shaft (2) is made of Nickel Titanum alloy, the cutting head (3) is made of stainless steel and the interlayer (4) is made of pure Tantalum.

## Patentansprüche

1. Eine flexible intramedulläre Reibahle (1) umfassend einen mit einer Formgedächtnislegierung gefertigten flexiblen Schaft (2) und einen zusammengeschweißten Schneidkopf (3), **dadurch gekennzeichnet, dass** der flexible Schaft (2) mittels einer mit Tantal gefertigten Zwischenschicht (4) mit dem Schneidkopf (3) zusammengeschweißt ist.

2. Eine flexible intramedulläre Reibahle (1) gemäß Anspruch 1, **dadurch gekennzeichnet, dass** der flexible Schaft (2) mit einer Nickel-Titan-Legierung gefertigt ist, der Schneidkopf (3) mit korrosionsbeständigem Stahl gefertigt ist und die Zwischenschicht (4) mit reinem Tantal gefertigt ist.

3. Eine flexible intramedulläre Reibahle (1) gemäß Anspruch 1 oder Anspruch 2, **dadurch gekennzeichnet, dass** der flexible Schaft (2), die Zwischenschicht (4) und der Schneidkopf (3) hohle Röhren sind, die ein Durchgangsloch (20) des flexiblen Schafts (2), ein Durchgangsloch (40) der Zwischenschicht (4) und ein Durchgangsloch (30) des Schneidkopfs (3) umfassen, die zueinander ausgerichtet sind und dieselben Maße haben, die für einen Führungsdraht angepasst sind, der geeignet ist, hindurchzupassen.

4. Verfahren zum Zusammenfügen eines mit einer Formgedächtnislegierung gefertigten flexiblen Schafts (2) und eines Schneidkopfs (3) einer flexiblen intramedullären Reibahle (1), **dadurch gekennzeichnet, dass** es eine Schweißphase umfasst, um den flexiblen Schaft (2) mit dem Schneidkopf (3) mittels einer mit Tantal gefertigten Zwischenschicht (4) zu schweißen.

5. Verfahren gemäß Anspruch 4, **dadurch gekennzeichnet, dass** die Schweißphase zumindest zwei Schritte umfasst,
wobei ein erster Schweißschritt dazu da ist, einen ersten Endabschnitt (42) der Zwischenschicht (4) mittels Rotationsreibschweißen mit einem Endabschnitt (21) des flexiblen Schafts (2) zu schweißen, und
wobei ein zweiter Schweißschritt dazu da ist, einen zweiten Endabschnitt (41) der Zwischenschicht (4) mittels Rotationsreibschweißen mit einem Endabschnitt (32) des Schneidkopfs (3) zu schweißen.

6. Verfahren gemäß Anspruch 5, **dadurch gekennzeichnet, dass**
der erste Schweißschritt eine erste geschweißte Gruppe (2, 4), die aus dem mit der Zwischenschicht (4) zusammengeschweißten flexiblen Schaft (2) besteht, bildet und
der zweite Schweißschritt dem ersten Schweißschritt nachfolgt.

7. Verfahren gemäß Anspruch 5 oder Anspruch 6, **dadurch gekennzeichnet, dass**
vor dem ersten Schweißschritt ein erster Vorbereitungsschritt vorgesehen ist, um eine Oberfläche des ersten Endabschnitts (42) der Zwischenschicht (4) flach zu machen und eine Oberfläche des Endabschnitts (21) des flexiblen Schafts (2) flach zu machen, und
vor dem zweiten Schweißschritt ein zweiter Vorbereitungsschritt vorgesehen ist, um eine Oberfläche des zweiten Endabschnitts (41) der Zwischenschicht (4) flach zu machen und eine Oberfläche des Endabschnitts (32) des Schneidkopfs (3) flach zu machen.

8. Verfahren gemäß Anspruch 7, **dadurch gekennzeichnet, dass** nach dem ersten Vorbereitungsschritt ein erster Reinigungsschritt vorgesehen ist, um mit einer Dekontaminationslösung die Oberfläche des ersten Endabschnitts (42) der Zwischenschicht (4) und die Oberfläche des Endabschnitts (21) des flexiblen Schafts (2) zu reinigen, und
nach dem zweiten Vorbereitungsschritt ein zweiter Reinigungsschritt vorgesehen ist, um mit der Dekontaminationslösung die Oberfläche des zweiten Endabschnitts (41) der Zwischenschicht (4) und die Oberfläche des Endabschnitts (32) des Schneidkopfs (3) zu reinigen.

9. Verfahren gemäß einem der vorstehenden Ansprüche 5-8, **dadurch gekennzeichnet, dass**
nach dem ersten Schweißschritt ein erster Entfernungsschritt vorgesehen ist, und
nach dem zweiten Schweißschritt ein zweiter Entfernungsschritt vorgesehen ist,
wobei der erste Entfernungsschritt und der zweite Entfernungsschritt geeignet sind, durch mechanische Mittel Schweißgrate von Schweißabschnitten der flexiblen intramedullären Reibahle (1) zu entfernen.

10. Verfahren gemäß einem der vorstehenden Ansprüche 4-9, **dadurch gekennzeichnet, dass** der flexible Schaft (2) mit einer Nickel-Titan-Legierung gefertigt ist, der Schneidkopf (3) mit korrosionsbeständigem Stahl gefertigt ist und die Zwischenschicht (4) mit reinem Tantal gefertigt ist.

## Revendications

1. Alésoir intramédullaire flexible (1) comprenant un arbre flexible (2) réalisé avec un alliage à mémoire de forme et une tête de coupe (3) soudés ensemble, **caractérisé en ce que** ledit arbre flexible (2) est soudé conjointement avec ladite tête de coupe (3) au moyen d'une couche intermédiaire (4) réalisée à partir de tantale.

2. Alésoir intramédullaire flexible (1) selon la revendication 1, **caractérisé en ce que** l'arbre flexible (2) est réalisé à partir d'alliage de nickel titane, la tête de coupe (3) est réalisée à partir d'acier inoxydable et la couche intermédiaire (4) est réalisée à partir de tantale pur.

3. Alésoir intramédullaire flexible (1) selon les revendications 1 ou 2, **caractérisé en ce que** l'arbre flexible (2), la couche intermédiaire (4) et la tête de coupe (3) sont des tubes creux comprenant un trou débouchant (20) de l'arbre flexible (2), un trou débouchant (40) de la couche intermédiaire (4) et un trou débouchant (30) de la tête de coupe (3) alignés ensemble et ayant les mêmes mesures adaptées pour qu'un filguide approprié passe à travers ces derniers.

4. Procédé pour assembler un arbre flexible (2) réalisé à partir d'un alliage mémoire de forme et une tête de coupe (3) d'un alésoir intramédullaire flexible (1), **caractérisé en ce qu'**il comprend une phase de soudage pour souder ledit arbre flexible (2) avec ladite tête de coupe (3) au moyen d'une couche intermédiaire (4) réalisée à partir de tantale.

5. Procédé selon la revendication 4, **caractérisé en ce que** ladite phase de soudage comprend au moins deux étapes,
une première étape de soudage consistant à souder une première partie d'extrémité (42) de ladite couche intermédiaire (4) au moyen du soudage par friction rotative avec une partie d'extrémité (21) dudit arbre flexible (2), et
une seconde étape de soudage consistant à souder une seconde partie d'extrémité (41) de ladite couche intermédiaire (4) au moyen du soudage par friction rotative avec une partie d'extrémité (32) de ladite tête de coupe (3).

6. Procédé selon la revendication 5, **caractérisé en ce que** :
ladite première étape de soudage forme un premier groupe soudé (2, 4) comprenant ledit arbre flexible (2) soudé conjointement avec ladite couche intermédiaire (4), et
ladite seconde étape de soudage est successive à ladite première étape de soudage.

7. Procédé selon les revendications 5 ou 6, **caractérisé en ce que** :
avant la première étape de soudage, on prévoit une première étape préparatoire consistant à aplanir une surface de la première partie d'extrémité (42) de ladite couche intermédiaire (4) et à aplanir une surface de la partie d'extrémité (21) dudit arbre flexible (2), et
avant la seconde étape de soudage, on prévoit une seconde étape préparatoire consistant à aplanir une surface de la seconde partie d'extrémité (41) de ladite couche intermédiaire (4) et à aplanir une surface de la partie d'extrémité (32) de ladite tête de coupe (3).

8. Procédé selon la revendication 7, **caractérisé en ce qu'**après la première étape préparatoire, on prévoit une première étape de nettoyage consistant à nettoyer, avec une solution de décontamination, la surface de la première partie d'extrémité (42) de ladite couche intermédiaire (4) et la surface de la partie d'extrémité (21) dudit arbre flexible (2), et
après la seconde étape préparatoire, on prévoit une seconde étape de nettoyage consistant à nettoyer, avec ladite solution de décontamination, la surface de la seconde partie d'extrémité (41) de ladite couche intermédiaire (4) et la surface de la partie d'extrémité (32) de ladite tête de coupe (3).

9. Procédé selon l'une quelconque des revendications 5 à 8, **caractérisé en ce que** :
après la première étape de soudage, on prévoit une première étape de retrait, et
après la seconde étape de soudage, on prévoit une seconde étape de retrait,
lesdites première et seconde étapes de retrait sont appropriées pour retirer, par des moyens mécaniques, les bavures de soudure des parties de soudure de l'alésoir intramédullaire flexible (1).

10. Procédé selon l'une quelconque des revendications 4 à 9, **caractérisé en ce que** l'arbre flexible (2) est réalisé avec un alliage de nickel titane, la tête de coupe (3) est réalisée à partir d'acier inoxydable et la couche intermédiaire (4) est réalisée à partir de tantale pur.
